(19) Europäisches Patentamt
European Patent Office
Office européen des brevets

(11) Numéro de publication: **0 606 795 A1**

(12) **DEMANDE DE BREVET EUROPEEN**

(21) Numéro de dépôt: **93403053.7**

(22) Date de dépôt: **16.12.93**

(51) Int. Cl.5: **C07D 207/412**, C23F 11/14, C07D 413/06

(30) Priorité: **11.01.93 FR 9300161**

(43) Date de publication de la demande: **20.07.94 Bulletin 94/29**

(84) Etats contractants désignés: **AT BE CH DE ES GB IT LI NL SE**

(71) Demandeur: **SOCIETE FRANCAISE HOECHST**
**TOUR ROUSSEL HOECHST**
**1 Terrasse Bellini**
**F-92800 Puteaux(FR)**

(72) Inventeur: **Wilhelm, Didier**
**8, sentier des Tricots**
**FR-92130 Issy les Moulineaux(FR)**
Inventeur: **Soreau, Michel**
**1, quai Galtier Boissière**
**F-77130 Barbizon(FR)**
Inventeur: **Blanc, Alain**
**61, avenue Romain Rolland**
**F-93200 Saint Denis(FR)**

(74) Mandataire: **Rinuy, Santarelli**
**14, avenue de la Grande Armée**
**F-75017 Paris (FR)**

(54) **Succinimides substituées, leur procédé de préparation et leur application comme inhibiteur de corrosion.**

(57) Succinimides substitués de formule (I)

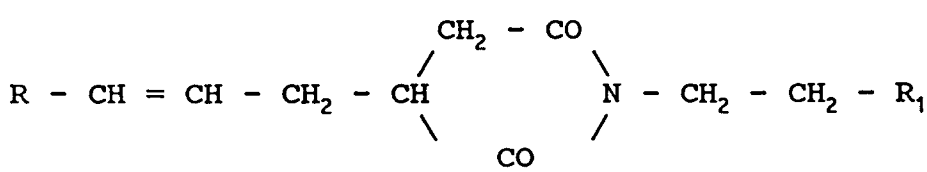

dans laquelle R représente un radical alcoyle contenant de 5 à 15 atomes de carbone et $R_1$ représente un radical oxo-2 morpholino ou un radical (carboxyméthyl)(hydroxy-2 éthyl) amino et leurs sels, et utilisation pour l'obtention de concentrats destinés au travail des métaux , notamment pour lutter contre la corrosion.

EP 0 606 795 A1

La présente demande concerne des succinimides substituées, leur procédé de préparation et leur application comme inhibiteur de corrosion.

Dans le domaine du travail des métaux, on utilise couramment des inhibiteurs de corrosion en solution ou en suspension dans des systèmes généralement aqueux. Comme inhibiteur de corrosion, il est connu d'employer des sels de métaux alcalins ou d'alcanolamine de divers acides minéraux ou organiques tels que le nitrite de sodium, le phosphate dipotassique, le métaborate de barium, le benzoate de sodium, des acides amiques libres ou salifiés tels que les acides succinamiques ou maléamiques substitués (cf. Kirk-Othmer, Encyclopedia of Chemical Technology, Vol. 7, page 137, 3ème édition, John Wiley and Sons, New York, 1984, brevets des Etats Unis d'Amérique N° 3 394 145, 3 646 151, 4 053 426, 4 130 433, 4 148 605, 4 235, 874, 4 326 987, 4 332 737, 4 737 159, demandes de brevet européen N° 106 234, 127 132, 191 952, 216 280, 359 048, 464 473, 501 318). Tous ces produits quoique actifs présentent toutefois un certain nombre d'inconvénients. En effet, aujourd'hui on recherche des inhibiteurs de corrosion actifs, atoxiques, facilement recyclables, aisément dégradables, économiques, hydrosolubles ou dispersibles dans l'eau, stables vis à vis des sels de dureté de l'eau de ville, respectant l'environnement et ne présentant aucun risque pour l'utilisateur.

Or les inhibiteurs de corrosion connus ne répondent pas entièrement aux souhaits des utilisateurs, notamment au niveau de leur coût.

Afin de satisfaire ces besoins, la demanderesse a découvert avec étonnement de nouvelles succinimides substituées présentant d'intéressantes propriétés anti-corrosives.

La présente invention a pour objet des produits de formule (I) :

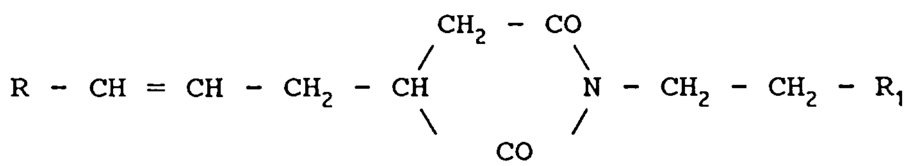

(I)

dans laquelle R représente un radical alcoyle contenant de 5 à 15 atomes de carbone et $R_1$ représente un radical oxo-2 morpholino ou un radical (carboxyméthyl)(hydroxy-2 éthyl) amino, sous leurs formes cis et/ou trans, racémiques ou optiquement actives, ainsi que les sels de métaux alcalins, alcalino-terreux ou d'amines des produits de formule (I) dans laquelle R a la signification déjà donnée et $R_1$ représente un radical (carboxyméthyl)(hydroxy-2 éthyl) amino.

Le terme alcoyle contenant de 5 à 15 atomes de carbone peut désigner, par exemple, un radical pentyle, hexyle, heptyle, octyle, nonyle, décyle, undécyle, dodécyle, tridécyle, tétradécyle, pentadécyle.

Les sels d'amines des produits de formule (I) dans laquelle R a la signification déjà donnée et $R_1$ représente un radical (carboxyméthyl)(hydroxy-2 éthyl) amino sont les sels d'amines usuelles. Parmi les amines usuelles, on peut citer, par exemple, les alcanolamines telles que la triéthanolamine, la monoéthanolamine. Les sels de métaux alcalins des produits de formule (I) dans laquelle R a la signification déjà donnée et $R_1$ représente un radical (carboxyméthyl)(hydroxy-2 éthyl) amino sont de préférence ceux de sodium ou de potassium.

L'invention a plus particulièrement pour objet les produits tels que définis ci-dessus, caractérisés en ce que dans la formule (I) R représente un radical N-pentyle, n-nonyle ou n-pentadécyle et $R_1$ a la signification déjà donnée précédemment, sous leurs formes cis et/ou trans racémiques ou optiquement actives, ainsi que les sels de métaux alcalins, alcalino-terreux ou d'amines des produits de formule (I) dans laquelle R a la signification déjà donnée et $R_1$ représente un radical méthyle (carboxyméthyl) (hydroxy-2 éthyl) amino.

Parmi ces derniers produits, on peut citer plus particulièrement :

- l'(octène-2 yl)-3 N-[(oxo-2 morpholino)-2 éthyl] succinimide,
- le (dodécène-2 yl)-3 N-[(oxo-2 morpholino)-2 éthyl] succinimide,
- l'(octodécène-2 yl)-3 N-[(oxo-2 morpholino)-2 éthyl] succinimide, et
- la N-(hydroxy-2 éthyl) N-[((octène-2 yl)-3 succinimido)-2 éthyl] glycine,
- la N-(hydroxy-2 éthyl) N-[((dodécène-2 yl)-3 succinimido)-2 éthyl] glycine,
- la N-(hydroxy-2 éthyl) N-[((octodécène-2 yl)-3 succinimido)-2 éthyl] glycine ainsi que leurs sels de métaux alcalins, alcalino-terreux, de monoéthanolamine et/ou de triéthanolamine.

Selon l'invention, les produits de formule (I) ci-dessus et leurs sels peuvent être préparés par un procédé caractérisé en ce que l'on fait réagir du glyoxal sur le produit de formule (II) :

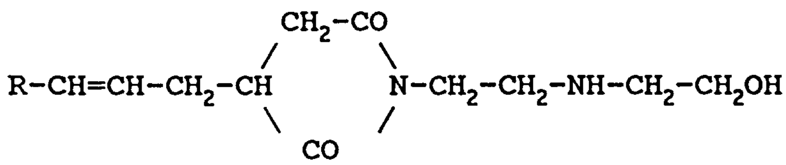

(II)

dans laquelle R a la signification déjà donnée précédemment pour obtenir un produit de formule $I_A$ correspondant à un produit de formule I dans laquelle R a la signification déjà indiquée et $R_1$ représente un radical (oxo-2 morpholino) :

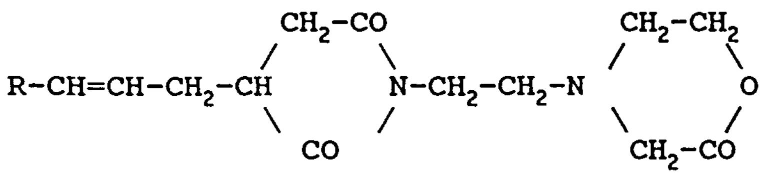

($I_A$)

que soit l'on isole, soit, si désiré, l'on hydrolyse par exemple par simple chauffage en milieu aqueux, pour obtenir une produit de formule ($I_B$) correspondant à un produit de formule (I) dans laquelle R a la signification déjà indiquée et $R_1$ représente un radical ((carboxyméthyl)(hydroxy-2 éthyl) amino).

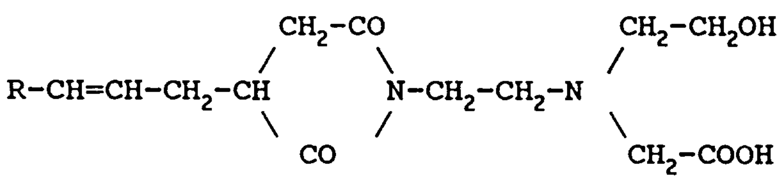

($I_B$)

que l'on isole et, si désiré, l'on salifie selon les méthodes usuelles.

Les produits de formule (II) sont des produits connus ou qui peuvent être aisément obtenus par condensation de la N-(hydroxy-2 éthyl) éthylènediamine sur l'anhydride succinique substitué correspondant de formule (III) dans laquelle R a la signification déjà donnée (cf. demande de brevet européen N° 417.990).

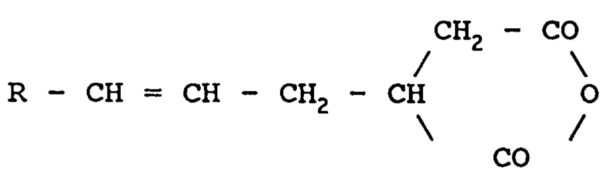

(III)

Les produits de formule (III) sont pour la plupart des produits commerciaux et ils sont facilement préparés par réaction de l'anhydride maléique avec l'alcène de formule (IV) :

$R - CH_2 - CH = CH_2$ (IV)

dans laquelle R a la signification déjà donnée.

Dans des conditions préférentielles de mise en oeuvre, le procédé ci-dessus décrit est réalisé de la manière suivante :

- la condensation du glyoxal avec le produit de formule (II) est effectuée à une température comprise entre 50 et 80 °C, en employant jusqu'à 25 % d'excès de glyoxal en solution aqueuse à 40 % en poids,
- l'hydrolyse du produit de formule ($I_A$) est réalisée par simple chauffage dans 3 à 5 parties en poids d'eau à une température comprise entre 40 et 100 °C.

L'invention a également pour objet des concentrats contenant un ou plusieurs produits de formule (I) ci-dessus en solution soit dans de l'eau soit dans de l'eau contenant éventuellement un monoalcoyle éther d'un glycol tel que le diéthylèneglycol monobutyléther, désigné ci-après BDG, soit dans une émulsion eau dans huile.

Ces concentrats contiennent outre un ou plusieurs produits de formule (I) ci-dessus à une concentration pondérale supérieure ou égale à 20 %, un ou plusieurs agents basiques choisis dans le groupe constitué par l'hydroxyde de sodium, l'hydroxyde de potassium, la monoéthanolamine, désignée ci-après MEA, la triéthanolamine, désignée ci-après TEA. Des exemples de tels concentrats sont donnés dans le tableau I dans lequel la nature du produit de formule (I) utilisé, référencé par son numéro donné dans les exemples est mentionné à la ligne A, son poids exprimé en produit tel quel est donné à la ligne B et son poids exprimé en produit sec est donné à la ligne C ; à la ligne MA sont mentionnés les poids exprimés en sec du produit de formule (I) et des divers agents basiques utilisés. L'huile H1 est une huile commerciale dont la répartition des carbones selon la norme ASTM D 2140 est Ca = 12,3 %, Cp = 47,3 % et Cn = 40,4 %, de densité 0,9 et d'indice de réfraction 1,4936 à 20 °C. Les expressions KOH à 50 % et NaOH à 50 % signifient de l'hydroxyde de potassium ou de l'hydroxyde de sodium à 50 % en poids dans l'eau. A la ligne pH sont indiquées les valeurs de pH d'une solution aqueuse contenant 5 % en poids de MA dans de l'eau de ville. Toutes les quantités mentionnées dans le tableau I sont exprimées en grammes.

La quantité d'agent(s) basique(s) introduit(s) dans le concentrat selon la présente invention est déterminée de telle façon qu'après sa dilution avec de l'eau de ville à une concentration de 5 % en poids de MA, le pH de la solution aqueuse obtenue soit compris entre 7 et 10, avantageusement entre 9 et 10.

Les produits de formule (I) ci-dessus sous leurs formes cis, trans, racémiques et/ou optiquement actives ainsi que les sels de métaux alcalins, alcalino-terreux ou d'amines des produits de formule (I) dans laquelle R a la signification déjà donnée et $R_1$ représente un radical (hydroxy-2 éthyl)(carboxyméthyl) amino présentent d' intéressantes propriétés anticorrosives.

Ces propriétés justifient l'application des succinimides substitués ci-dessus ainsi que de leurs sels pour l'obtention de concentrats destinés au travail des métaux et à l'inhibition de la corrosion des métaux.

Ces propriétés sont notamment mises en lumière par le test décrit dans la norme DIN 51360, blatt II, en utilisant des copeaux de fonte grise de référence GG25. Les résultats de ce test effectué au départ de divers concentrats selon la présente invention après leur dilution avec de l'eau de ville à une concentration pondérale en MA de 1 à 5 % sont donnés dans le tableau II, dans lequel en fonction de la concentration en MA des concentrats C1-C10, sont mentionnées des notes de 0 à 5 ; 0 indiquant une corrosion nulle et 5 une corrosion importante.

L'examen de ce tableau permet de constater que les concentrats C4-C6 à une concentration de 2% en MA dans de l'eau de ville protège très efficacement la fonte contre la corrosion. Dans ce test le produit de formule (I) qui est le plus actif est la N-(hydroxy-2 éthyl) N-[((octène-2 yl)-3 succinimido)-2 éthyl] glycine.

Les spectres RMN$^{13}$C des produits décrits dans les exemples ont été enregistrés avec un appareil BRUCKER AC100 à 25 MHz ou à 50 MHz.

Les déplacements chimiques indiqués ne concernent que certains atomes de carbone à l'exclusion des atomes de carbone de la chaîne R - CH = CH - $CH_2$ - et des atomes de carbone méthine et méthylène du cycle succinimide.

Les exemples suivants illustrent la présente invention sans toutefois la limiter.

**EXEMPLE 1**

(octène-2 yl)-3 N[(oxo-2 morpholino)-2 éthyl] succinimide.

**Stade 1.**

On introduit lentement, sous agitation vigoureuse, en maintenant la température du milieu réactionnel à 70 °C, 1 mole (104,15 g) de N-(hydroxy-2 éthyl)éthylènediamine dans 1 mole (210,26 g) d'anhydride (octène-2 yl)-1 succinique. L'introduction terminée, on poursuit l'agitation pendant une heure à 70 °C, puis 50 minutes à 150 °C tout en distillant sous pression réduite l'eau formée.

Après refroidissement du milieu réactionnel à 20 °C, on obtient 296 g d'(octène-2 yl)-3 N[(hydroxy-2 éthyl)amino-2 éthyl] succinimide, 11 (II avec R = n-pentyle) sous la forme d'une huile.

**Analyses**

| $C_{16}H_{28}N_2O_3$ | |
|---|---|
| 296,39 | N% 9,3 (théorie 9,45) |
| Dosage d'eau (K.Fisher) : 0,3 % | |

RMN$^{13}$C(DMSO, $d_6$, ppm) : 179,5 ; 176,6 ; 60,3 ; 51,2 ; 46,5 ; 37,9.

**Stade 2.**

On introduit en 30 minutes, sous agitation, en maintenant la température du milieu réactionnel à 80 °C, 98 g d'une solution aqueuse de glyoxal à 40 % en poids, soit 0,676 mole, dans 200 g (0,675 mole) de 11 préparé à l'exemple 1, stade 1. L'introduction terminée, on poursuit l'agitation pendant 2 heures à 80 °C, puis on élimine l'eau formée par distillation sous pression réduite. Après refroidissement du milieu réactionnel à 20 °C, on obtient 225 g (0,67 mole) d'(octène-2 yl)-3 N[(oxo-2 morpholino)-2 éthyl]-succinimide, 12 (($I_A$)avec R = n-pentyle) sous forme d'une huile très épaisse.

**Analyses**

| $C_{16}H_{28}N_2O_4$ | |
|---|---|
| 336,41 | N% 8,2 (théorie 8,33) |
| Dosage d'eau (K.Fisher) : 0,7 % | |

RMN$^{13}$C(DMSO, $d_6$, ppm) : 179,4 ; 176,5 ; 166,9 ; 68,5 ; 55,1 ; 53,1 ; 47,0 ; 34,9.
Cette huile distille à 220-230 °C sous une pression de 50Pa.

**EXEMPLE 2**

N-(hydroxy-2 éthyl) N-[(octène-2 yl)-3 succinimido)-2 éthyl] glycine.

On chauffe 3 heures sous agitation à 40 °C, puis une heure à 65 °C, et enfin 1 heure à l'ébullition, 50,5 g (149 mmoles) de 12 préparé à l'exemple 1, stade 2, dans 150 g d'eau distillée. Après refroidissement du milieu réactionnel à la température ambiante, on obtient 200 g d'une solution aqueuse contenant 52,8 g (149 mmoles) de N-(hydroxy-2 éthyl) N-[(octène-2 yl)-3 succinimido)-2 éthyl] glycine, 13, (($I_B$) avec R = n-pentyle)).

Cette solution aqueuse contient 73,7 % d'eau déterminée par dosage selon K.Fisher, 2,1 % d'azote et un dosage acidimétrique donné 0,64 meq/g (théorie 0,74 meq/g).

**Analyses**

RMN$^{13}$C(DMSO, $d_6$, ppm) : 181 ; 178,2 ; 171,3 ; 58,1 ; 56,6 ; 56,3 ; 52,1 ; 35,6.

**EXEMPLE 3**

(dodécène-2 yl)-3 N[oxo-2 morpholino)-2 éthyl] succinimide.

**Stade 1.** On reproduit le stade 1 de l'exemple 1 au départ de 1 mole d'anhydride (dodécène-2 yl)-1 succinique et 1 mole de N-(hydroxy-2 éthyl) éthylènediamine. On obtient ainsi 352g de(dodécène-2 yl)-3 N-[(hydroxy-2 éthyl) amino-2 éthyl] succinimide, 21, ((II) avec R = n-nonyle) sous la forme d'une huile.

**Analyses**

| $C_{20}H_{36}N_2O_3$ | |
|---|---|
| 352,51 | N% 7,6 (théorie 7,95) |
| Dosage d'eau (K.Fisher) : 0,55 % | |

RMN$^{13}$C(DMSO, $d_6$, ppm) : 180 ; 178 massif ; 176,5 ; 60,2 ; 51,1; 46,1 ; 37,9.

Cette huile est uitlisée telle quelle pour le stade 2.

**Stade 2.** On reproduit le stade 2 de l'exemple 1 au départ de 0,675 mole (239 g) de 21 préparé au stade 1 ci-dessus et de 0,675 mole de glyoxal en solution aqueuse à 40 % en poids. On obtient ainsi 278 g de (dodécène-2 yl)-3 N[oxo-2 morpholino)-2 éthyl] succinimide, 22, (($I_A$) avec R = n-nonyle)) sous la forme d'une huile.

**Analyses**

| $C_{22}H_{36}N_2O_4$ | |
|---|---|
| 392,52 | N% 6,8 (théorie 7,14) |
| Dosage d'eau (K.Fisher) : 4,9 % | |

RMN$^{13}$C(DMSO, $d_6$, ppm) : 180-178 massif ; 176,3 ; 166,7 ; 68,5; 55,1 ; 53,2 ; 48 ; 34,7.

**EXEMPLE 4**

N-(hydroxy-2 éthyl) N-[((dodécène- 2yl)-3 succinimido)-2 éthyl] glycine.

On reproduit l'exemple 2 au départ de 128 mmoles de 22 préparé à l'exemple 2 stade 2 et de 149 g d'eau. On obtient ainsi 201,7 g d'une solution aqueuse colorée contenant 128 mmoles (52,5 g) de N-(hydroxy-2 éthyl) N-[((dodécène- 2yl)-3 succinimido)-2 éthyl] glycine, 23, (($I_B$) avec R = n-nonyle). Cette solution aqueuse contient 74 % d'eau déterminée par dosage selon K.Fisher, 1,9 % d'azote et le dosage acidimétrique est de 0,64 meq/g (théorie 0,64 meq/g).

**Analyse**

RMN$^{13}$C(acétone, $d_6$, ppm) : 182-178 massif ; 178 ; 170 ; 57,2; 56,7 ; 56,5 ; 53,1 ; 34,4.

**EXEMPLE 5**

(octadécène-2 yl)-3 N-[(oxo-2 morpholino)-2 éthyl] succinimide.

**Stade 1.** On reproduit le stade 1 de l'exemple 1 au départ de 1 mole d'anhydride (octadécène-2 yl)-1 succinique et 1 mole de N-(hydroxy-2 éthyl) éthylènediamine. On obtient ainsi 436 g d'(octadécène-2 yl)-3 N-[(hydroxy-2 éthyl) amino-2 éthyl] succinimide, 31, ((II) avec R = n-pentadécyle) sous la forme d'une huile.

**Analyses**

| | |
|---|---|
| $C_{26}H_{48}N_2O_3$ | |
| 436,66 | N% 6,8 (théorie 6,42) |
| Dosage d'eau (K.Fisher) : 1,4 % | |

RMN$^{13}$C(DMSO, $d_6$, ppm) : 177 ; 174,5 ; 60 ; 51 ; 47,5 (un signal est confondu avec celui du DMSO).

**Stade 2.** On reproduit le stade 2 de l'exemple 1 au départ de 0,675 mole (299 g) de 31 préparé au stade 1 ci-dessus et de 0,675 mole de glyoxal en solution aqueuse à 40 % en poids. On obtient ainsi 325,6 g d'-(octadécène-2 yl)-3 N-[(oxo-2 morpholino)-2 éthyl] succinimide, 32 (($I_A$) avec R = n-pentadécyle) sous forme d'une huile.

**Analyses**

| | |
|---|---|
| $C_{28}H_{48}N_2O_4$ | |
| 476,68 | N% 6,3 (théorie 5,88) |
| Dosage d'eau (K.Fisher) : 1,2 % | |

RMN$^{13}$C(DMSO, $d_6$, ppm) : 179,3 ; 176,4 ; 166,9 ; 68,5 ; 55,1 ; 53,2 ; 47,9 ; 35.

**EXEMPLE 6**

On reproduit l'exemple 2 au départ de 34 mmoles (16,4 g) de 32 préparé à l'exemple 3, stade 2 et de 45 g d'eau distillée. On obtient ainsi environ 61 g d'une pâte colorée contenant 34 mmoles (16,8 g) de N-(hydroxy-2 éthyl) N-[(octadécène-2 yl)-3 succinimido)-2 éthyl] glycine, 33, (($I_B$) avec R = n-pentadécyle).

**TABLEAU I**

**Compositions pondérales des concentrats**

| | C1 | C2 | C3 | C4 | C5 | C6 | C7 | C8 | C9 | C10 |
|---|---|---|---|---|---|---|---|---|---|---|
| A | 12 | 22 | 32 | 13 | 13 | 13 | 13 | 23 | 23 | 33 |
| B | 69 | 62,5 | 41,5 | 88 | 92,2 | 88,4 | 96 | 88,5 | 91,9 | 88,3 |
| C | 68,5 | 59,4 | 41 | 23,14 | 24,24 | 23,25 | 25,24 | 23,0 | 23,9 | 24,28 |
| MEA | 10,3 | 6,25 | 4,5 | 5,1 | | | | 4,3 | | 2,9 |
| TEA | 20,7 | 18,75 | 12,5 | 6,9 | | 7,0 | | 7,2 | | 8,8 |
| KOH à 50 % | | | | | 7,8 | | 4 | | 3,1 | |
| NaOH à 50 % | | | | | | 4,6 | | | | |
| M.A | 99,5 | 84,4 | 58 | 35,14 | 28,14 | 32,55 | 27,24 | 34,5 | 25,45 | 35,98 |
| Eau de ville | | 12,5 | | | | | | | | |
| Huile H1 | | | 41,5 | | | | | | | |
| BDG | | | | | | | | | 5 | |
| Poids Total | 100 | 100 | 100 | 100 | 100 | 100 | 100 | 100 | 100 | 100 |
| pH | 9,2 | 9,1 | 9,0 | 9,2 | 9,1 | 9,0 | 7,1 | 9,3 | 7,25 | 9,3 |

**TABLEAU II**

**Résultats des essais de corrosion en fonction de la dilution de divers concentrats.**

| Concentrats / Concentrations | C1 | C2 | C3 | C4 | C5 | C6 | C7 | C8 | C9 | C10 |
|---|---|---|---|---|---|---|---|---|---|---|
| 1 % | | | | | 4 | 4 | | | | |
| 1,5 % | | | | 4 | 1 | 4 | 4 | | | |
| 2 % | | 3 | | 0 | 0 | 0 | | 2 | 4 | |
| 2,5 % | | | | | 0 | 0 | | | | |
| 3 % | | | | | | | | | 4 | |
| 3,5 % | | | | | | | | | | |
| 4 % | | | 1 | | | | 1 | | 4 | 1 |
| 4,5 % | | | | | | | | | | |
| 5 % | 2 | 0 | 0 | 0 | | | 0 | 0 | 4 | 1 |

## Revendications

**1.** Succinimides substitués de formule (I)

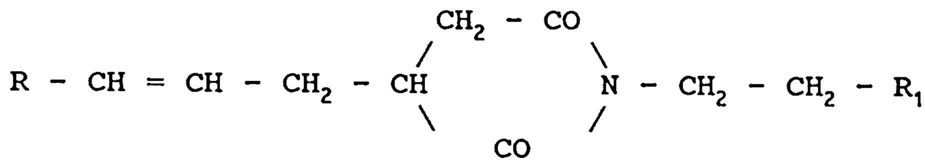

dans laquelle R représente un radical alcoyle contenant de 5 à 15 atomes de carbone et $R_1$ représente un radical oxo-2 morpholino ou un radical (carboxyméthyl)(hydroxy-2 éthyl) amino.

**2.** Sels de métaux alcalins, alcalino-terreux ou d'amine des succinimides telles que définies à la revendication 1 caractérisées par le fait que dans la formule (I), R représente un radical alcoyle contenant de 5 à 15 atomes de carbone et $R_1$ représente un radical (carboxyméthyl)(hydroxy-2 éthyl) amino.

**3.** (Octène-2 yl)-3 N-[(oxo-2 morpholino)-2 éthyl] succinimide.

**4.** (Dodécène-2 yl)-3 N-[(oxo-2 morpholino)-2 éthyl] succinimide.

**5.** (Octodécène-2 yl)-3 N-[(oxo-2 morpholino)-2 éthyl] succinimide.

**6.** N-(hydroxy-2 éthyl) N-[((dodécène-2 yl)-3 succinimido)-2 éthyl] glycine ou ses sels de métaux alcalins, alcalino-terreux, de monoéthanolamine ou de triéthanolamine.

**7.** N-(hydroxy-2 éthyl) N-[((octène-2 yl)-3 succinimido)-2 éthyl] glycine ou ses sels de métaux alcalins, alcalino-terreux, de monoéthanolamine ou de triéthanolamine.

**8.** N-(hydroxy-2 éthyl) N-[((octadécène-2 yl)-3 succinimido)-2 éthyl] glycine ou ses sels de métaux alcalins, alcalino-terreux, de monoéthanolamine ou de triéthanolamine.

**9.** Sels selon la revendication 2 caractérisés par le fait que ce sont des sels d'amines choisies dans le groupe constitué par la monoéthanolamine et la triéthanolamine.

**10.** Sels selon la revendication 2 caractérisés par le fait que ce sont des sels de métaux alcalins choisis dans le groupe constitué par le sodium et le potassium.

**11.** Utilisation des succinimides substitués ou de leurs sels tels que définis à l'une quelconque des revendications 1 à 10 pour l'obtention de concentrats destinés au travail des métaux.

Office européen des brevets

# RAPPORT DE RECHERCHE EUROPEENNE

Numero de la demande
EP 93 40 3053

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | US-A-3 762 873 (OUDE ALINK) 2 Octobre 1973<br>* colonne 6, ligne 1 - ligne 10 *<br>* exemple 5 * | 1-11 | C07D207/412<br>C23F11/14<br>C07D413/06 |
| A | EP-A-0 417 990 (ETHYL PETROLEUM ADDITIVES) 20 Mars 1991<br>* RN 133937-80-1, 2,5-Pyrrolidinedione, 1-[2-[(2-hydroxyethyl)amino]ethyl]-3-(2-octylidenedodecyl)- * | 1-11 | |
| A | US-A-3 903 005 (KABLAOUI, M. S. ET. AL.) 2 Septembre 1975<br>* le document en entier * | 1-11 | |
| A | EP-A-0 026 878 (HOECHST AG) 15 Avril 1981<br>* le document en entier * | 1-11 | |
| A | EP-A-0 191 952 (HOECHST AG) 27 Août 1986<br>* le document en entier * | 1-11 | |
| A | GB-A-1 373 411 (BRITISH PETROLEUM COMPANY LTD.) 13 Novembre 1974<br>* le document en entier * | 1-11 | DOMAINES TECHNIQUES RECHERCHES (Int.Cl.5)<br>C07D |

Le présent rapport a été établi pour toutes les revendications

| Lieu de la recherche | Date d'achèvement de la recherche | Examinateur |
|---|---|---|
| LA HAYE | 11 Avril 1994 | Kissler, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)